# EUROPEAN PATENT APPLICATION

(11) **EP 3 482 691 A1**
(43) Date of publication of application: **15.05.2019**
(21) Application number: 17201574.5
(22) Date of filing: 14.11.2017
(51) Int. Cl.: A61B 8/12, A61B 8/08, A61B 8/00, G01S 15/89

(54) **ICE CATHETER WITH MULTIPLE TRANSDUCER ARRAYS**

(71) Applicant: Koninklijke Philips N.V., 5656 AE Eindhoven (NL)
(72) Inventor: KOLEN, Alexander Franciscus, 5656 AE Eindhoven (NL); JANSE, Cornelis Pieter, 5656 AE Eindhoven (NL); BELT, Harm Jan Willem, 5656 AE Eindhoven (NL); SCHMEITZ, Harold Agnes Wilhelmus, 5656 AE Eindhoven (NL); VAN HEESCH, Franciscus Hendrikus, 5656 AE Eindhoven (NL); SMEETS, Bart, 5656 AE Eindhoven (NL)
(74) Representative: de Haan, Poul Erik

(57) **Abstract**

An ultrasound image compounding method that is suitable for use with an ICE catheter that includes a first ultrasound transceiver array and a second ultrasound transceiver array, the first ultrasound transceiver array and the second ultrasound transceiver array being axially separated along a length of the ICE catheter, is described. In the method, first array data corresponding to ultrasound signals detected by the first ultrasound transducer array in response to an insonification of a region of interest by the first ultrasound transducer array at a first insonification angle; and second array data corresponding to ultrasound signals detected by the second ultrasound transducer array in response to an insonification of the region of interest by the second ultrasound transducer array at a second insonification angle; are received. A compound image corresponding to the region of interest is generating based on the first array data and the second array data.

## Description

### FIELD OF THE INVENTION

The invention relates to an ultrasound image compounding method for use with an Intra Cardiac Echography, i.e. ICE, catheter. The invention finds application in the field of cardiac medical procedures.

### BACKGROUND OF THE INVENTION

ICE is widely used during interventional cardiac procedures to visualize anatomical features such as the atrial septum, the aortic valve, pulmonary veins and so forth. ICE is also used to image interventional devices such as ablation catheters and lasso catheters that are used in performing medical procedures on the heart. An ICE catheter includes an array of ultrasound transducer elements which is used to generate an ultrasound image. Conventionally, ICE catheters use a one-dimensional array of ultrasound elements to generate a two-dimensional image slice. Such two-dimensional image slices have a somewhat limited field of view, and so ICE catheters that use a two-dimensional array of ultrasound elements to generate a three-dimensional, i.e. volumetric, image have also been developed.

Ultrasound images are in general susceptible to image artifacts. Speckle is one such artifact that appears as randomly fluctuating bright and dark image pixels. Speckle arises from the manner in which ultrasound echoes from an object's surface are processed in an ultrasound imaging system. The reflections from a number of scatterers on the object surface are coherently added, allowing random fluctuations in the phase of these reflections to translate as an amplitude noise, thereby degrading image quality.

Another common ultrasound image artifact is shadowing. Shadowing occurs particularly in two-dimensional ultrasound images and results in darker image regions behind acoustically-dense image features. Shadowing arises from the reduced ultrasound signal propagating beyond such image features.

Image compounding refers to a group of techniques that are used in the ultrasound field to reduce such image artifacts by combining different images of a region of interest; see for instance the document entitled "Multi-Angle Compound Imaging" by Jespersen, S. K. et al., Ultrasonic Imaging, Vol. 20, pages 81 - 102, 1998. One form of image compounding termed "spatial compounding" involves the insonification of a region of interest at multiple incidence angles and combining the information into a single image. The combining of images from multiple angles reduces both shadowing, and also the variance of the speckle. Another form of image compounding termed "frequency compounding" that is described in more detail in a document "Phasing out speckle" by Gatenby, J. C. et al, Phys Med Biol. 1989 Nov, 34(11), pages 1683-9 involves the combining of image data that is generated at multiple insonification frequencies into a single image. The speckle patterns from each frequency are thereby averaged and the resulting speckle is likewise reduced. Frequency compounding has been used alone or in combination with spatial compounding.

A document entitled "A probabilistic framework for freehand 3D ultrasound reconstruction applied to catheter ablation guidance in the left atrium" by Koolwal, A. B., et al., Int. J. CARS (2009) 4:425-437 describes the use of an ICE catheter to collect 2D-ICE images of a left atrium phantom from multiple configurations and iteratively compound the acquired data into a 3D-ICE volume. Two methods for compounding overlapping ultrasound data are described: occupancy likelihood and response-grid compounding, which automatically classify voxels as "occupied" or "clear," and mitigate reconstruction artifacts caused by signal dropout.

In spite of these developments there remains room to improve ICE images.

### SUMMARY OF THE INVENTION

The present invention seeks to reduce speckle in ICE imaging procedures. Further advantages from the described invention will also be apparent to the skilled person. Thereto an ultrasound image compounding method, a computer program product, an ICE catheter and an ultrasound imaging arrangement are presented.

The ultrasound image compounding method is suitable for use with an ICE catheter that includes a first ultrasound transceiver array and a second ultrasound transceiver array in which the first ultrasound transceiver array and the second ultrasound transceiver array are axially separated along a length of the ICE catheter. The method includes the steps of: i) receiving, from the first ultrasound transceiver array, first array data corresponding to ultrasound signals detected by the first ultrasound transducer array in response to an insonification of a region of interest by the first ultrasound transducer array at a first insonification angle; ii) receiving, from the second ultrasound transceiver array, second array data corresponding to ultrasound signals detected by the second ultrasound transducer array in response to an insonification of the region of interest by the second ultrasound transducer array at a second insonification angle; and iii) generating, based on the first array data and the second array data, a compound image corresponding to the region of interest.

In so doing, at least some of the first array data and at least some of the second array data both correspond to the same region of interest; i.e. they correspond to an overlapping region. By so combining the data generated by the two axially-separated transducer arrays, the resulting compound image, i.e. a combined image that has been generated by insonification of the same region of interest at multiple insonification angles, has reduced speckle in this region of interest. This is because the speckle patterns from each array, being based on detected ultrasound signals from two insonification angles of the region of interest, are averaged in the compound image, i.e. combined image, which reduces the variance of the speckle. Moreover the two different insonification angles of the region of interest result in less shadowing behind acoustically-dense image features as compared to an ICE catheter that uses only a single viewing angle or a single array. The use of two axially-separated transducer arrays also advantageously increases the range of insonification angles that the region of interest can be subjected to, thereby further reducing shadowing. By reducing these image artifacts an improved ultrasound image is provided. In some implementations the separate arrays may be operated simultaneously. This reduces the time to scan the region of interest across the desired angular range and thereby reduces image blurring caused by intra-scan ICE catheter motion. In other implementations, when the two arrays are included on a flexible catheter, a bend in the catheter between the two arrays can be used to further reduce shadowing since this also increases the range of insonification angles that the region of interest can be subjected to.

In accordance with one aspect the first ultrasound transceiver array and the second ultrasound transceiver array have a mutual spatial arrangement. In this aspect the step of generating the compound image is based further on the mutual spatial arrangement.

The mutual spatial arrangement aspect may include, for example, the steps of receiving, from the first ultrasound transceiver array, first array tracking data corresponding to ultrasound signals detected by the first ultrasound transducer array in response to ultrasound signals emitted by the second ultrasound transducer array; or receiving, from the second ultrasound transceiver array, second array tracking data corresponding to ultrasound signals detected by the second ultrasound transducer array in response to ultrasound signals emitted by the first ultrasound transducer array; and determining the mutual spatial arrangement based on the first array tracking data or the second array tracking data correspondingly. In so doing, an ultrasound transceiver array's tracking data is provided by ultrasound signals that are emitted by the other of the two ultrasound transceiver arrays. The provision of tracking data in this manner, i.e. using ultrasound signals, provides a simple means of determining the mutual spatial arrangement because the existing ultrasound transceiver arrays are used. The mutual spatial arrangement may be determined by computing a distance between the two ultrasound transceiver arrays, the distance being computed based on a time of flight of the ultrasound signals. In one implementation the ultrasound signals may be dedicated tracking signals that are specifically directed towards the opposite ultrasound transceiver array. In another implementation the ultrasound signals may be stray ultrasound signals emitted by the other ultrasound transceiver array, for example a sidelobe of the opposite ultrasound transceiver array's insonification of the region of interest. In another implementation the ultrasound signals emitted by each ultrasound transducer array in the mutual spatial arrangement aspect may form a hemispherical wave front that radiates outwardly. In this last implementation a receiving ultrasound transducer array's tracking data may correspond to time of flight data indicative of a distance between the transmitting array and each of a plurality of array elements of the receiving ultrasound transceiver array. The mutual spatial arrangement may subsequently be determined based on the receiving ultrasound transducer array's tracking data by triangulating the position of each of the plurality of array elements of the receiving ultrasound transceiver array respective the transmitting array.

Alternatively the mutual spatial arrangement aspect may include the use of data from a bend sensor or a bend actuator on the ICE catheter, the bend sensor or actuator being configured to provide bend data indicative of a bend of the catheter between the first ultrasound transceiver array and the second ultrasound transceiver array. In this aspect the method further includes the step of receiving the bend data and determining the mutual spatial arrangement based on a model configured to predict the mutual spatial arrangement based on the bend data. Advantageously this aspect provides a reliable compound image because the mutual positions of the two ultrasound transducer arrays are accurately determined.

Alternatively the mutual spatial arrangement aspect may include the use of a first ultrasound transceiver array that is a two-dimensional array for generating a volumetric ultrasound image. In this aspect the mutual spatial arrangement is determined by: reconstructing a volumetric ultrasound image based on the first array data, the volumetric ultrasound image comprising a plurality of two-dimensional image slices; reconstructing a planar ultrasound image based on the second array data; and matching one of the plurality of two-dimensional image slices to the planar ultrasound image based on at least one image feature in the region of interest. Advantageously this aspect does not require additional sensors to determine the mutual spatial arrangement.

In accordance with another aspect an ICE catheter for use with any of the aforementioned methods is presented. The ICE catheter includes a first ultrasound transceiver array and a second ultrasound transceiver array in which the first ultrasound transceiver array and the second ultrasound transceiver array are axially separated along the length of the ICE catheter. The first ultrasound transceiver array and the second ultrasound transceiver array each include an array surface configured to transmit and to receive ultrasound signals. Preferably the array surface of the first ultrasound transceiver array and the array surface of the second ultrasound transceiver array are mutually parallel in a rotational direction about the length axis, or more specifically: mutually parallel in a direction that is tangential to the circumference of a virtual circle that is formed by rotating a point perpendicularly about the length axis. This aspect helps to improve the size of the region of interest, i.e. the overlapping insonified region. Optionally the ICE catheter may include a bend sensor or a bend actuator.

In accordance with another aspect an ultrasound imaging arrangement is presented. The ultrasound imaging arrangement includes an ICE catheter including a first ultrasound transceiver array and a second ultrasound transceiver array. The first ultrasound transceiver array and the second ultrasound transceiver array are axially separated along the length of the ICE catheter. The ultrasound imaging arrangement also includes a processor configured to execute any of the aforementioned methods.

Further aspects are described with reference to the appended claims.

### BRIEF DESCRIPTION OF THE DRAWINGS

Fig. 1 illustrates a prior art form of spatial compounding in which an ultrasound transducer array 110 insonifies an acoustically dense object 111 from multiple directions.
Fig. 2 illustrates a first embodiment of the invention in which an ICE catheter 220 includes a first ultrasound transceiver array 210₁ and a second ultrasound transceiver array 210₂.
Fig. 3 illustrates a method that may be used with the ICE catheter of Fig. 1.
Fig. 4 illustrates an embodiment of an ICE catheter 220 in which the mutual spatial arrangement of first ultrasound transceiver array 210₁ and second ultrasound transceiver array 210₂ is determined from ultrasound signals detected by an ultrasound transducer array in response to ultrasound signals emitted by the other ultrasound transducer array.
Fig. 5 illustrates an embodiment of an ICE catheter 220 that includes a bend sensor 215.
Fig. 6 illustrates an ultrasound imaging arrangement 600 that includes ICE catheter 220 and a processor 622.

### DETAILED DESCRIPTION OF EMBODIMENTS

In order to illustrate the principles of the present invention an ultrasound image compounding method is described with particular reference to an ICE catheter that is used to image cardiac structures. It is however to be appreciated that the ultrasound image compounding method also finds application with an ICE catheter that is used to image interventional devices such as an ablation catheter, a lasso catheter used for electrophysiological mapping of cardiac structures, and so forth. Moreover an ICE catheter is described that includes a first and a second ultrasound transducer array. The relative positions of the arrays, with the first ultrasound transducer array illustrated as being at the distal end of the catheter should not be interpreted as limiting. For example the relative positions of these arrays may be reversed such that the second ultrasound transducer array is at the distal end, or indeed the arrays may be positioned at other positions along the length of the ICE catheter than the distal end.

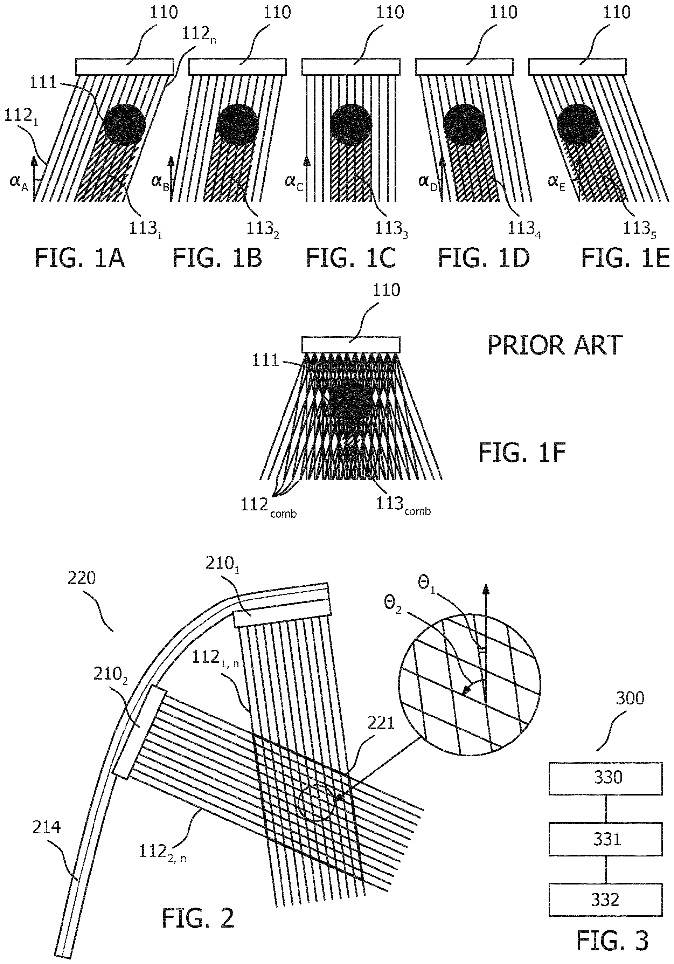

ICE is widely used during interventional cardiac procedures to visualize anatomical features such as the atrial septum, the aortic valve, pulmonary veins and so forth. As with other conventional ultrasound imaging systems, ICE suffers from two common image artifacts: speckle and shadow. One technique that is used in the ultrasound field to mitigate these artifacts is image compounding. Spatial compounding is a particular form of image compounding that involves the insonification of a region of interest at several incidence angles and combining the information into a single image. See for instance document "Multi-Angle Compound Imaging" by Jespersen, S. K. et al., Ultrasonic Imaging, Vol. 20, pages 81 - 102, 1998. In this regard, Fig. 1 illustrates a prior art form of spatial compounding in which an ultrasound transducer array 110 insonifies an acoustically dense object 111 from multiple directions. In the series of figures 1A..1E, image scan lines 112_{i=1..n} that insonify acoustically dense object 111 from each of several different angles α_{A..E} are generated using beam steering techniques by applying relative delays to the ultrasound signals emitted by and detected by the plurality of array elements that form ultrasound transducer array 110. By adjusting the insonification angle with respect to acoustically dense object 111, a different acoustic shadow 113_{i=1..n} is generated at each insonification angle α. The acoustic shadow is a region of lower intensity ultrasound signals that results from the blocking of ultrasound signals by acoustically dense object 111. In an ultrasound B-mode image, image features in this region tend to appear darker than would be expected owing to the reduced intensity ultrasound signals in this region. In the prior art technique of spatial compounding a combined image is generated by performing a weighted sum of the ultrasound image data, i.e. intensity, obtained at corresponding positions in the region of interest from each insonification angle. As illustrated in Fig. IF, the combined effect of all image scan lines 112_{comb} is to reduce the combined shadow 113_{comb}. A secondary effect of this weighting is a reduction in speckle. This results from the averaging of the speckle patterns from each angle α, wherein the averaging reduces the variance of the speckle.

Frequency compounding is another particular form of image compounding. Frequency compounding involves the insonification of the object at several frequencies and combining the information into a single image. See for instance the document "Phasing out speckle" by Gatenby, J. C., et al, Phys. Med. Biol., 1989, Vol. 34, No. 11, 1683 - 1689.

With reference to Fig. 1, the combination of frequency compounding and spatial compounding additionally involves, prior to generating the combined image: band pass filtering, at each of a plurality of central frequencies, the temporal ultrasound signals corresponding to each of the plurality of image scan lines 112_{i=1..n}, and computing for each image scan line, an weighted average of the band pass filtered ultrasound signals. Consequent to the weighting of the contributions from each frequency in the combined image, frequency compounding may further reduce speckle.

Fig. 2 illustrates a first embodiment of the invention in which an ICE catheter 220 includes a first ultrasound transceiver array 210₁ and a second ultrasound transceiver array 210₂. ICE catheter 220 includes a length axis 214. First ultrasound transceiver array 210₁ and second ultrasound transceiver array 210₂ are axially separated along the length of ICE catheter 220. The first ultrasound transceiver array and the second ultrasound transceiver array each include an array surface configured to transmit and to receive ultrasound signals. Preferably the array surface of the first ultrasound transceiver array and the array surface of the second ultrasound transceiver array are mutually parallel in a rotational direction about length axis 214, or more specifically: mutually parallel in a direction that is tangential to the circumference of a virtual circle that is formed by rotating a point perpendicularly about length axis 214. This helps to improve the size of region of interest 221, i.e. the overlapping insonified region. Fig. 3 illustrates a method that may be used with the ICE catheter of Fig. 2. With reference to Fig. 3 and Fig. 2, the method includes the steps of: receiving 330, from first ultrasound transceiver array 210₁, first array data corresponding to ultrasound signals detected by first ultrasound transducer array 210₁ in response to an insonification of region of interest 221 by first ultrasound transducer array 210₁ at first insonification angle θ₁; receiving 331, from second ultrasound transceiver array 210₂, second array data corresponding to ultrasound signals detected by second ultrasound transducer array 210₂ in response to an insonification of region of interest 221 by second ultrasound transducer array 210₂ at second insonification angle θ₂; and generating 332, based on the first array data and the second array data, a compound image corresponding to region of interest 221.

By so combining the data generated by axially-separated transducer arrays 210₁, 210₂, the resulting spatially-compounded image has reduced speckle. The speckle patterns from each array are averaged in the compound image, thereby reducing the variance of the speckle. Moreover the different insonification angles of the region of interest result in less shadowing behind acoustically-dense image features as compared to an ICE catheter that uses a single viewing angle or a single array. By reducing these image artifacts an improved ultrasound image is provided. The use of axially-separated transducer arrays 210₁, 210₂ advantageously increases the range of insonification angles θ₁, θ₂ that region of interest 221 can be subjected to, thereby further reducing shadowing. Moreover, the separate arrays 210₁, 210₂ may be operated simultaneously. This reduces the time to scan region of interest 221 across the desired angular range and thereby reduces image blurring caused by intra-scan motion of ICE catheter 220. When a flexible catheter is used for ICE catheter 220 a bend in ICE catheter 220 between the two arrays 210₁, 210₂ can be used to further reduce shadowing since this bend further increases the range of insonification angles that the region of interest can be subjected to. Such a flexible catheter is also termed a steerable catheter and may include a control unit configured to provide a desired bend. This facilitates e.g. the steering of the catheter around chambers in the heart.

Whilst an idealized array of parallel image scan lines 112_{1,n} and 112_{2,n} are illustrated in Fig. 2, it is to be appreciated that alternative beam patterns may be used. Such beam patterns may exemplarily include beam patterns that have a beam waist, beams that are generated using a subset array elements of the ultrasound transducer arrays, or beams that are steered using known beamforming techniques in directions other than the perpendicular directions with respect to arrays 210₁, 210₂ that are illustrated in Fig. 2. Clearly such alternative beams must, as illustrated in Fig. 2, likewise overlap at region of interest 221.

ICE catheter 220 in Fig. 2 is illustrated as including a bend between ultrasound transducer arrays 210₁, 210₂, however other shaped catheters including for example a straight catheter or a bendable or steerable catheter may alternatively be used.

Ultrasound transducer arrays 210₁, 210₂ may be one-dimensional or two-dimensional arrays, each including a plurality of array elements. Thereto the arrays may, correspondingly be used to generate and detect ultrasound signals corresponding to either a planar or a volumetric, i.e. 3D ultrasound images. Moreover, more than two ultrasound transducer arrays may be included on catheter 220 in the same manner. In one embodiment described later, one of the arrays is a two-dimensional array and another of the arrays is either a one- or a two-dimensional array. The array elements may include for example piezoelectric material or may be membrane-based i.e. Capacitive Micro machined Ultrasonic Transducers, a technology referred-to as CMUT.

In accordance with one implementation the method step of generating 332 includes: weighting the first array data and the second array data at corresponding positions in the region of interest 221; and summing the weighted first array data and the weighted second array data at the corresponding positions to provide the compound image. In some instances equal weighting of the first and second array data may be used. Differing weighting values may alternatively be used.

In accordance with one implementation the method step of generating 332 includes: reconstructing a first ultrasound image based on the first array data, the first ultrasound image including first ultrasound image intensity data; reconstructing a second ultrasound image based on the second array data, the second ultrasound image including second ultrasound image intensity data; weighting the first ultrasound image intensity data and the second ultrasound image intensity data at corresponding positions in the region of interest 221; and summing the weighted first ultrasound image intensity data and the weighted second ultrasound image intensity data at the corresponding positions to provide the compound image. One of many known ultrasound image reconstruction techniques may be used, for example to reconstruct brightness, or B-mode ultrasound images that include the ultrasound image intensity data. Although other weighting factors may be used, preferably a weighting is used in which each of the two ultrasound images contribute equally to corresponding positions in the compound image.

In accordance with another implementation the method step of generating 332 includes: comparing the first array data and the second array data at corresponding positions in region of interest 221 and selecting the largest value of said data at each corresponding position to provide the compound image. This form of image compounding offers improved boundary definition at the expense of somewhat lowered speckle reduction.

In accordance with another implementation, frequency compounding may be used in combination with the above-described techniques of spatial compounding. Thereto, in this implementation the first array data includes temporal ultrasound signals corresponding to each of a plurality of image scan lines at the first insonification angle 112_{1,n}; and the second array data includes temporal ultrasound signals corresponding to each of a plurality of image scan lines at the second insonification angle 112_{2,n}. Moreover, the step of generating a compound image corresponding to the region of interest 221 includes: band pass filtering, at each of a plurality of central frequencies, the temporal ultrasound signals corresponding to each of a plurality of image scan lines at the first insonification angle 112_{1,n} and computing for each image scan line, a weighted average of the band pass filtered ultrasound signals; band pass filtering, at each of a plurality of central frequencies, the temporal ultrasound signals corresponding to each of a plurality of image scan lines at the second insonification angle 112_{2,n} and computing for each image scan line, a weighted average of the band pass filtered ultrasound signals; and summing the weighted average of the band pass filtered ultrasound signals at the first insonification angle 112_{1,n} and the weighted average of the band pass filtered ultrasound signals at the second insonification angle 112_{2,n} at corresponding positions in the region of interest 221 to provide the compound image. In so doing, additional speckle reduction may be achieved.

In accordance with another implementation, first ultrasound transceiver array 210₁ and second ultrasound transceiver array 210₂ have a mutual spatial arrangement. In this implementation the step of generating 332 is based further on the mutual spatial arrangement. In so doing, any changes in the mutual spatial arrangement can be accounted-for in the generated compound image.

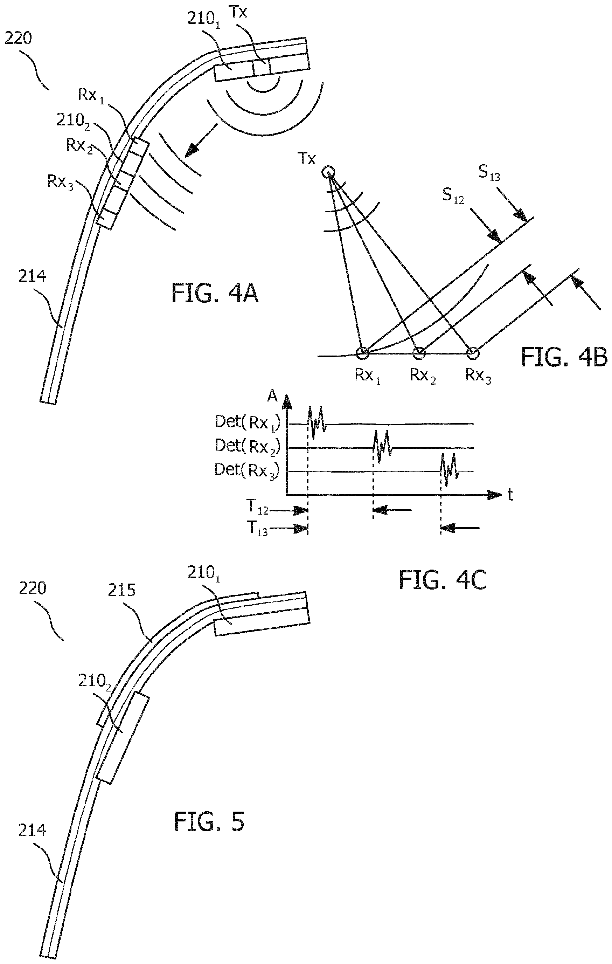

In one embodiment of this mutual spatial arrangement implementation, array tracking data is generated by one or both of the ultrasound transceiver arrays 210₁, 210₂. In this embodiment an ultrasound transceiver array's tracking data is provided by ultrasound signals that are emitted by the other of the two ultrasound transceiver arrays. Thereto, the method may include the steps of: receiving, from first ultrasound transceiver array 210₁, first array tracking data corresponding to ultrasound signals detected by the first ultrasound transducer array 210₁ in response to ultrasound signals emitted by the second ultrasound transducer array 210₂; or receiving, from the second ultrasound transceiver array 210₂, second array tracking data corresponding to ultrasound signals detected by the second ultrasound transducer array 210₂ in response to ultrasound signals emitted by the first ultrasound transducer array 210₁; and determining the mutual spatial arrangement based on the first array tracking data or the second array tracking data correspondingly. An ultrasound transducer array's emitted signals may be dedicated tracking pulses, or indeed stray ultrasound signals such as a sidelobe of those that are used by that ultrasound transducer array to insonify the region of interest. The latter has the benefit of simplifying the control of the ultrasound signals emitted by an array because it involves detecting the other ultrasound transducer array's stray imaging signals. Such sidelobes are typically present as a result of the beamforming techniques that are used to insonify region of interest 221 at the respective insonification angle θ₁, θ₂. The benefit of using dedicated tracking pulses is that these may advantageously be directed towards the expected positon of the other, receiving, array, thereby minimizing the emitted ultrasound power. One example implementation of this mutual spatial arrangement is illustrated in Fig. 4, which illustrates an embodiment of an ICE catheter 220 in which the mutual spatial arrangement of first ultrasound transceiver array 210₁ and second ultrasound transceiver array 210₂ is determined from ultrasound signals detected by an ultrasound transducer array in response to ultrasound signals emitted by the other ultrasound transducer array.

In Fig. 4A and Fig. 4B, first ultrasound transceiver array 210₁ operates as an emitter with a transmitter element Tx and second ultrasound transceiver array 210₂ operates as a detector with multiple detector elements Rx_{1..3}. As illustrated in Fig. 4B and Fig. 4C, ultrasound signals in the form of an ultrasound pulse emitted by transmitter Tx impinge upon detectors Rx_{1..3}. The pulse is detected by detectors Rx_{1..3} as illustrated by signals Det(Rx_{1..3}) and the times of flight of each of the ultrasound signals correspond to the distances between transmitter Tx and the corresponding receiver Rx_{1..3}. Differences T₁₂, T₁₃, in these times of flight corresponds to differences S₁₂, S₁₃ in these distances. The mutual spatial arrangement of first ultrasound transceiver array 210₁ and second ultrasound transceiver array 210₂ can subsequently be calculated via triangulation based on these distances. Clearly the same effect may also be achieved by reversing the functionality of first ultrasound transceiver array 210₁ and second ultrasound transceiver array 210₂ such that first ultrasound transceiver array 210₁ operates as a detector. Although triangulation based on three distances is illustrated in Fig 4, in some cases three distances are not required; for example when the bending of ICE catheter 220 is constrained such that it can only bend in one dimension. In this case a single distance measurement may suffice.

As illustrated in Fig. 4A, the ultrasound signals emitted by first ultrasound transducer array 210₁ may form a hemispherical wave front radiating outwardly with respect to the first ultrasound transducer array. Such a wave front may be beneficial when ICE catheter 220 has significant bending freedom owing to its relatively uniform power distribution in directions away from first ultrasound transducer array 210₁. Alternatively, beam steering techniques may be used to generate a more directed beam that is directed more specifically at the opposite transducer array in order to reduce the power required from the ultrasound signals. As an alternative to the dedicated ultrasound signals of this and the hemispherical wave front example, as mentioned above, stray ultrasound signals, i.e. a side lobe of the other array's insonification of the region of interest may be detected in the same manner.

Fig. 5 illustrates an embodiment of an ICE catheter 220 that includes a bend sensor 215. Bend sensor 215 may be used to determine the mutual spatial arrangement of first ultrasound transceiver array 210₁ and second ultrasound transceiver array 210₂ as an alternative to the above-described ultrasound signals. Bend sensor 215 extends between first ultrasound transceiver array 210₁ and second ultrasound transceiver array 210₂. Bend sensor 215 may for example be a strain gauge such as a fiber Bragg grating, or a capacitive position sensor or another type of bend sensor altogether. In so doing, bend sensor 215 provides bend data indicative of a bend of ICE catheter 220 between first ultrasound transceiver array 210₁ and second ultrasound transceiver array 210₂. The bend data may be used in combination with a model that describes the mutual spatial arrangement of first ultrasound transceiver array 210₁ and second ultrasound transceiver array 210₂ based on the bend data, to determine the mutual spatial arrangement. In another configuration that is not illustrated, ICE catheter 220 may include a bend actuator configured to provide a desired bend and corresponding bend data indicative of a bend of ICE catheter 220 between first ultrasound transceiver array 210₁ and second ultrasound transceiver array 210₂. In the same way, the bend data may be used in combination with a model that describes the mutual spatial arrangement of first ultrasound transceiver array 210₁ and second ultrasound transceiver array 210₂ based on the bend data, to determine the mutual spatial arrangement. A known type of actuator for use in such a steerable catheter uses wires that extend within the catheter, which when provided with a predetermined tension provide a desired catheter bend. Upon releasing the tension the catheter relaxes back to its original form. Such models may for example relate a predetermined bend or ICE catheter tip displacement to empirical strain measurements.

In another embodiment that is described with reference to Fig. 2, instead of using the above-described bend data and instead of using the above-described ultrasound signals to determine the mutual spatial arrangement of first ultrasound transceiver array 210₁ and second ultrasound transceiver array 210₂, the mutual spatial arrangement may be determined by matching ultrasound images generated by each ultrasound transceiver array. In this embodiment, first ultrasound transceiver array 210₁ is a two-dimensional array for generating a volumetric ultrasound image. The mutual spatial arrangement is determined by: reconstructing a volumetric ultrasound image based on the first array data, the volumetric ultrasound image comprising a plurality of two-dimensional image slices; reconstructing a planar ultrasound image based on the second array data; and matching one of the plurality of two-dimensional image slices to the planar ultrasound image based on at least one image feature in the region of interest. Various known image matching algorithms may be used to perform the matching. Scale-invariant feature transform, SIFT, is one suitable example. Rigid or non-rigid image transforms known from the medical image processing field may also be used. The correlation technique described in US patent 5,655,535, or the real-time image alignment procedure described in US patent 8,303,505 may alternatively be used. Clearly the same effect may be achieved by also making the second ultrasound transceiver array 210₁ a two-dimensional array for generating a volumetric ultrasound image. In this regard, both of the arrays 210₁, 210₂ may be two-dimensional arrays in which one of the arrays is configured to generate a volumetric image and the other is configured to generate a planar image.

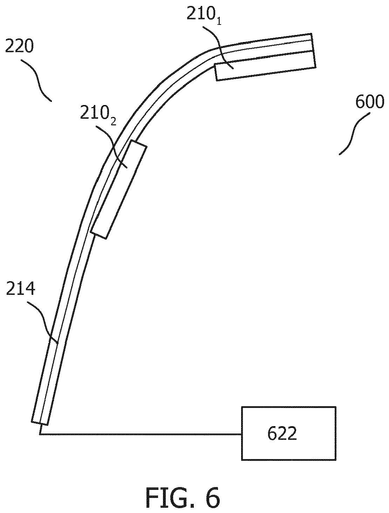

Any of the method steps presented herein, may be recorded in the form of instructions which when executed on a processor cause the processor to carry out such method steps. Thereto, Fig. 6 illustrates an ultrasound imaging arrangement 600 that includes ICE catheter 220 and a processor 622. Data transfer between ultrasound transducer arrays 210₁, 210₂ and processor 622 is illustrated by way of the link between these items. It is to be appreciated that either wired or wireless data communication is contemplated for this link. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD.

In summary, an ultrasound image compounding method has been described that is suitable for use with an ICE catheter that includes a first ultrasound transceiver array and a second ultrasound transceiver array, the first ultrasound transceiver array and the second ultrasound transceiver array being axially separated along a length of the ICE catheter. In the method, first array data corresponding to ultrasound signals detected by the first ultrasound transducer array in response to an insonification of a region of interest by the first ultrasound transducer array at a first insonification angle; and second array data corresponding to ultrasound signals detected by the second ultrasound transducer array in response to an insonification of the region of interest by the second ultrasound transducer array at a second insonification angle; are received. A compound image corresponding to the region of interest is generated based on the first array data and the second array data.

Various embodiments and implementations have been described in the above and it is noted that these may be combined to achieve further advantageous effects.

## Claims

1. An ultrasound image compounding method (300) for use with an ICE catheter (220) including a first ultrasound transceiver array (210₁) and a second ultrasound transceiver array (210₂) in which the first ultrasound transceiver array (210₁) and the second ultrasound transceiver array (210₂) are axially separated along a length of the ICE catheter (220); the method (300) comprising the steps of:
receiving (330), from the first ultrasound transceiver array (210₁), first array data corresponding to ultrasound signals detected by the first ultrasound transducer array (210₁) in response to an insonification of a region of interest (221) by the first ultrasound transducer array (210₁) at a first insonification angle (θ₁);
receiving (331), from the second ultrasound transceiver array (210₂), second array data corresponding to ultrasound signals detected by the second ultrasound transducer array (210₂) in response to an insonification of the region of interest (221) by the second ultrasound transducer array (210₂) at a second insonification angle (θ₂);
generating (332), based on the first array data and the second array data, a compound image corresponding to the region of interest (221).

2. The ultrasound image compounding method according to claim 1 wherein the generating (332) includes:
weighting the first array data and the second array data at corresponding positions in the region of interest (221); and
summing the weighted first array data and the weighted second array data at the corresponding positions to provide the compound image.

3. The ultrasound image compounding method according to claim 1 wherein the generating (332) includes:
reconstructing a first ultrasound image based on the first array data, the first ultrasound image including first ultrasound image intensity data;
reconstructing a second ultrasound image based on the second array data, the second ultrasound image including second ultrasound image intensity data;
weighting the first ultrasound image intensity data and the second ultrasound image intensity data at corresponding positions in the region of interest (221); and
summing the weighted first ultrasound image intensity data and the weighted second ultrasound image intensity data at the corresponding positions to provide the compound image.

4. The ultrasound image compounding method according to claim 1 wherein the generating (332) includes:
comparing the first array data and the second array data at corresponding positions in the region of interest (221) and selecting the largest value of said data at each corresponding position to provide the compound image.

5. The ultrasound image compounding method according to any one of claims 1 4 wherein:
the first array data includes temporal ultrasound signals corresponding to each of a plurality of image scan lines at the first insonification angle (112_{1,n});
the second array data includes temporal ultrasound signals corresponding to each of a plurality of image scan lines at the second insonification angle (112_{2,n});
wherein the generating (332) includes:
band pass filtering, at each of a plurality of central frequencies, the temporal ultrasound signals corresponding to each of a plurality of image scan lines at the first insonification angle (112_{1,n)} and computing for each image scan line, a weighted average of the band pass filtered ultrasound signals;
band pass filtering, at each of a plurality of central frequencies, the temporal ultrasound signals corresponding to each of a plurality of image scan lines at the second insonification angle (112_{2,n}) and computing for each image scan line, a weighted average of the band pass filtered ultrasound signals; and
summing the weighted average of the band pass filtered ultrasound signals at the first insonification angle (112_{1,n}) and the weighted average of the band pass filtered ultrasound signals at the second insonification angle (112_{2,n}) at corresponding positions in the region of interest (221) to provide the compound image.

6. The ultrasound image compounding method according to claim 1 wherein the first ultrasound transceiver array (210₁) and the second ultrasound transceiver array (210₂) have a mutual spatial arrangement; and wherein the step of generating (332) is based further on the mutual spatial arrangement.

7. The ultrasound image compounding method according to claim 6 further comprising the steps of:
receiving, from the first ultrasound transceiver array (210₁), first array tracking data corresponding to ultrasound signals detected by the first ultrasound transducer array (210₁) in response to ultrasound signals emitted by the second ultrasound transducer array (210₂); or
receiving, from the second ultrasound transceiver array (210₂), second array tracking data corresponding to ultrasound signals detected by the second ultrasound transducer array (210₂) in response to ultrasound signals emitted by the first ultrasound transducer array (210₁); and
determining the mutual spatial arrangement based on the first array tracking data or the second array tracking data correspondingly.

8. The ultrasound image compounding method according to claim 7 wherein the ultrasound signals emitted by the second ultrasound transducer array (210₂) correspond to at least one sidelobe of the insonification of the region of interest (221) by the second ultrasound transducer array (210₂) at the second insonification angle (θ₂); or wherein the ultrasound signals emitted by the first ultrasound transducer array (210₁) correspond to at least one sidelobe of the insonification of the region of interest (221) by the first ultrasound transducer array at the first insonification angle (θ₁); correspondingly.

9. The ultrasound image compounding method according to claims 7 - 8 wherein the step of determining the mutual spatial arrangement based on the corresponding first array tracking data or the corresponding second array tracking data, comprises:
for the first array tracking data, computing at least one distance between the first ultrasound transceiver array (210₁) and the second ultrasound transceiver array (210₂) based on a time of flight of the ultrasound signals detected by the first ultrasound transducer array (210₁) in response to ultrasound signals emitted by the second ultrasound transducer array (210₂), and
comprises for the second array tracking data, computing at least one distance between the first ultrasound transceiver array (210₁) and the second ultrasound transceiver array (210₂) based on a time of flight of the ultrasound signals detected by the second ultrasound transducer array (210₂) in response to ultrasound signals emitted by the first ultrasound transducer array (210₁).

10. The ultrasound image compounding method according to claim 7 wherein:
the ultrasound signals emitted by the second ultrasound transducer array (210₂) form a hemispherical wave front radiating outwardly with respect to the second ultrasound transducer array (210₂), wherein the first array tracking data corresponds to time of flight data indicative of a distance between the second ultrasound transducer array (210₂) and each of a plurality of array elements of the first ultrasound transceiver array (210₁), and wherein the mutual spatial arrangement is determined based on the first array tracking data by triangulating the position of each of the plurality of array elements of the first ultrasound transceiver array (210₁) respective the second ultrasound transducer array (210₂); or
wherein the ultrasound signals emitted by the first ultrasound transducer array (Tx, 210₁) form a hemispherical wave front radiating outwardly with respect to the first ultrasound transducer array (Tx, 210₁), wherein the second array tracking data corresponds to time of flight data indicative of a distance between the first ultrasound transducer array (Tx, 210₁) and each of a plurality of array elements of the second ultrasound transceiver array (Rx_{1..3}, 210₂), and wherein the mutual spatial arrangement is determined based on the second array tracking data by triangulating the position of each of the plurality of array elements of the second ultrasound transceiver array (Rx_{1..3}, 210₂) respective the first ultrasound transducer array (Tx, 210₁); correspondingly.

11. The ultrasound image compounding method according to claim 6 wherein the ICE catheter (220) further includes either i) a bend sensor (215) such as a strain gauge such as a fiber Bragg grating, or a capacitive position sensor, the bend sensor (215) being configured to provide bend data indicative of a bend of the ICE catheter (220) between the first ultrasound transceiver array (210₁) and the second ultrasound transceiver array (210₂), or ii) a bend actuator configured to provide a desired bend and corresponding bend data indicative of a bend of the ICE catheter (220) between the first ultrasound transceiver array (210₁) and the second ultrasound transceiver array (210₂);
and wherein the method further comprises the step of receiving the bend data and determining the mutual spatial arrangement based on a model configured to predict the mutual spatial arrangement based on the bend data.

12. The ultrasound image compounding method according to claim 6 wherein the first ultrasound transceiver array (210₁) is a two-dimensional array for generating a volumetric ultrasound image, and wherein the mutual spatial arrangement is determined by:
reconstructing a volumetric ultrasound image based on the first array data, the volumetric ultrasound image comprising a plurality of two-dimensional image slices;
reconstructing a planar ultrasound image based on the second array data; and matching one of the plurality of two-dimensional image slices to the planar ultrasound image based on at least one image feature in the region of interest.

13. Computer program product comprising instructions which when executed on a processor are configured to cause the processor to carry out the method steps according to claim 1.

14. An ICE catheter (220) for use with the method according to claim 1, the ICE catheter including a first ultrasound transceiver array and a second ultrasound transceiver array in which the first ultrasound transceiver array and the second ultrasound transceiver array are axially separated along the length of the ICE catheter.

15. Ultrasound imaging arrangement (600) comprising:
an ICE catheter (220) including a first ultrasound transceiver array (210₁) and a second ultrasound transceiver array (210₂) in which the first ultrasound transceiver array (210₁) and the second ultrasound transceiver array (210₂) are axially separated along the length of the ICE catheter (220); and
a processor (622) configured to execute the method according to claim 1.
